# EUROPEAN PATENT APPLICATION

(11) **EP 4 437 970 A1**
(43) Date of publication of application: **02.10.2024**
(21) Application number: 22898526.3
(22) Date of filing: 21.11.2022
(51) Int. Cl.: A61B 8/12, A61B 17/3205, F16C 1/02

(54) **TWO-LAYER COIL STRUCTURE**

(30) Priority: 25.11.2021 JP 2021191463
(71) Applicant: Asahi Intecc Co., Ltd., Seto-shi, Aichi 489-0071 (JP)
(72) Inventor: HASHIMOTO Takaya, Seto-shi, Aichi 489-0071 (JP)
(74) Representative: Winter, Brandl - Partnerschaft mbB
(86) International application number: PCT/JP2022/043006
(87) International publication number: WO 2023/095743

(57) **Abstract**

A torque coil (two-layer coil structure) 10 of the disclosed embodiments includes an inner coil 11 formed by spirally winding metal wire 111, and an outer coil 12 arranged to be in close contact with the outer periphery of the inner coil 11 and formed by spirally winding metal wire 121. In the torque coil 10, a winding direction of the inner coil 11 and a winding direction of the outer coil 12 are opposite from each other, and when the torque coil 10 is twisted in the circumferential direction being the direction where the diameter of the inner coil 11 is increased, a change amount of the diameter of the outer coil 12 is larger than a change amount of the diameter of the inner coil 11. A length 1596 mm is set as one unit of each of the inner coil 11 and the outer coil 12, and the change amount d₁ of the diameter of the inner coil 11 when the inner coil 11 of the one unit length is twisted by 360° in the circumferential direction being the direction where the diameter of the inner coil 11 is increased, and the change amount d₂ of the diameter of the outer coil 12 when the outer coil 12 of the one unit length is twisted by 360° in the circumferential direction being the direction where the diameter of the outer coil 12 is decreased, satisfy the relation of -4.5 < d₁/d₂ < -1.6. Such a two-layer coil structure has high twisting rigidity, thus suppressing the occurrence of kinks due to rotational resistance.

## Description

### [Technical Field]

The disclosed embodiments relate to a two-layer coil structure.

### [Background Art]

Conventionally, there is known a multi-layer coil structure referred to as a torque coil or a drive shaft, which is provided in a medical catheter or the like and used to transmit a rotating motion of a hand operation part to the distal end portion. For example, Patent Literature 1 discloses the drive shaft 10 that is rotatably provided in the catheter sheath 2 of the ultrasonic catheter 1 and is formed by a multiplex/multilayer tightly wound coil made of metal wire of stainless or the like. Moreover, Patent Literature 2 discloses the hollow drive shaft 16 that is provided in the catheter sheath 18 of the acoustic imaging (ultrasonic imaging) catheter 10 and includes the inner coil 40 and the outer coil 42 of, which are formed of wound nitinol.

### [Citation List]

### [Patent Literature]

[Patent Literature 1] JP 2003-062072 A
[Patent Literature 2] JP H09-504214 A

### [Summary of Invention]

### [Technical Problem]

Such a two-layer coil structure is rotationally driven by a drive source such as a motor connected to a hand side, and there is a problem that if the two-layer coil structure is stuck in a catheter during operation of the catheter, for example, and the rotational resistance against the coil part is increased, kinks are easily occurred at the coil part. The two-layer coil structure with kinks may not be able to transmit rotation smoothly. Moreover, with the occurrence of kinks, lead wire connected to the inside of the two-layer coil structure may be broken, thereby disabling the catheter itself.

In view of such aspects, the disclosed embodiments aim at providing a two-layer coil structure having high twisting rigidity and suppressing the occurrence of kinks due to rotational resistance.

### [Solution to Problem]

In order to achieve the above-described object, the disclosed embodiment provides a two-layer coil structure, including: an inner coil that is formed by spirally winding metal wire; and an outer coil that is arranged to be in close contact with an outer periphery of the inner coil and formed by spirally winding metal wire, in which a winding direction of the inner coil and a winding direction of the outer coil are opposite from each other, when the two-layer coil structure is twisted in a circumferential direction of the two-layer coil structure being the direction where a diameter of the inner coil is increased, a change amount of the diameter of the inner coil is smaller than a change amount of a diameter of the outer coil, a length 1596 mm is set as one unit of each of the inner coil and the outer coil, and the change amount d₁ of the diameter of the inner coil when the inner coil of the one unit length is twisted by 360° in the circumferential direction being the direction where the diameter of the inner coil is increased, and the change amount d₂ of the diameter of the outer coil when the outer coil of the one unit length is twisted by 360° in the circumferential direction being the direction where the diameter of the outer coil is decreased, satisfy the relation of -4.5 < d₁/d₂ < -1.6 (Disclosed embodiment 1).

In such a disclosed embodiment (Disclosed embodiment 1), the winding direction of the inner coil and the winding direction of the outer coil are opposite from each other. Thus, when the two-layer coil structure is twisted in the circumferential direction being the direction where the diameter of the inner coil is increased, the diameter of the inner coil is increased, while the diameter of the outer coil is decreased. As a result, the outer coil and the inner coil are pressed against each other. Then, with the change amount of the diameter of the inner coil smaller than the change amount of the diameter of the outer coil, the outer coil strongly tightens the inner coil, so that the layers of the outer coil and the inner coil are brought into firm and close contact with each other. Thus, the twisting rigidity of the two-layer coil structure is enhanced, which suppresses the occurrence of kinks due to rotational resistance. Especially, in a case where a length 1596 mm is set as one unit of each of the inner coil and the outer coil, and the change amount d₁ of the diameter of the inner coil when the inner coil of the one unit length is twisted by 360° in the circumferential direction being the direction where the diameter of the inner coil is increased, and the change amount d₂ of the diameter of the outer coil when the outer coil of the one unit length is twisted by 360° in the circumferential direction being the direction where the diameter of the outer coil is decreased, satisfy the relation of -4.5 < d₁/d₂ < -1.6, the maximum torque force of two-layer coil structure is improved, thereby achieving the two-layer coil structure having more excellent twisting rigidity.

In the above-described disclosed embodiment (Disclosed embodiment 1), the inner coil is preferably formed by spirally winding 2 to 18 metal wires.

### [Advantageous Effects of Invention]

In the disclosed embodiments, it is possible to provide a two-layer coil structure having high twisting rigidity and suppressing the occurrence of kinks due to rotational resistance.

### [Brief Description of Drawings]

[Fig. 1] Fig. 1 is an explanatory view illustrating a structure of a torque coil according to one of the disclosed embodiments.
[Fig. 2] Fig. 2 is an explanatory view illustrating a structure of a shaft main body according to the disclosed embodiment.
[Fig. 3] Fig. 3 is a graph illustrating the relation between the ratio of change amounts in a radial direction of the inner coil and the outer coil and the maximum torque force and the twisting rigidity in torque coils of examples and modifications.

### [Description of Embodiments]

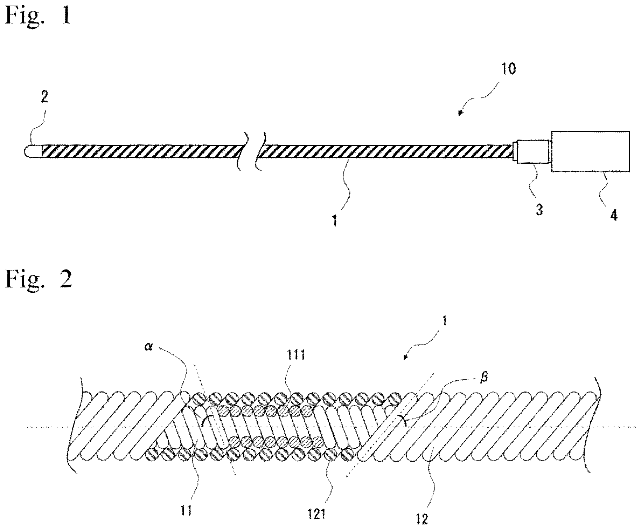

Hereinafter, the disclosed embodiments will be described with reference to the drawings. Fig. 1 is an explanatory view illustrating the entire structure of a torque coil 10 according to the embodiment, and Fig. 2 is an explanatory view illustrating a structure of a shaft main body 1 of the torque coil 10. Note that the disclosed embodiments are not limited to ones described in the following, which are merely examples to illustrate the technical features of the disclosed embodiments. Moreover, the shape and size in each drawing are merely illustrated to facilitate understanding of the contents of the disclosed embodiments, and do not precisely reflect the actual shape and size.

In the specification, the "distal end side" indicates a direction along the axial direction of the shaft main body 1 of the torque coil 10, the direction being a direction along which the torque coil 10 advances toward a treatment site. The "proximal end side" indicates a direction along the axial direction of the shaft main body 1 of the torque coil 10, the direction being a direction opposite to the above-described distal end side. Moreover, the "distal end" refers to an end on the distal end side of an arbitrary member or portion, and the "proximal end" refers to an end on the proximal end side of an arbitrary member or portion. Furthermore, the "distal end portion" refers to a portion, in an arbitrary member or portion, including the distal end and extending from the distal end toward the proximal end side to the halfway of the above-described member or the like, and the "proximal end portion" refers to a portion, in an arbitrary member or portion, including the proximal end and extending from the proximal end toward the distal end side to the halfway of the above-described member or the like. Note that in Fig. 1 and Fig. 2, the left side in the drawings is the "distal end side" to be inserted into a body in the state inserted in an ultrasonic catheter or the like, and the right side in the drawings is the "proximal end side" connected to a drive source such as a motor.

The torque coil 10 includes, as illustrated in Fig. 1, the long shaft main body 1, a housing 2 attached to the distal end side of the shaft main body 1, and a connector 3 attached to the proximal end side of the shaft main body 1. The shaft main body 1 of the embodiment has a hollow two-layer coil structure including, as illustrated in Fig. 2, an inner coil 11 formed by spirally winding metal wire 111 and an outer coil 12 arranged to be in close contact with the outer periphery of the inner coil 11 and formed by spirally winding metal wire 121.

The housing 2 is attached to the distal end of the shaft main body 1 of the torque coil 10. In a case where the torque coil 10 is used for an ultrasonic imaging catheter, a transducer (not illustrated) for ultrasonic imaging is built in the housing 2, and the housing 2 is attached to the distal end portion of the shaft main body 1 by a known fixing technique such as epoxy resin or an adhesive. Moreover, depending on the application of the torque coil 10, a coil member made of a different material from the shaft main body 1 or a connecting member for the connection to an operation object such as a medical clip may be joined instead of the housing 2, or the distal end portion of the shaft main body 1 may be brazed by a brazing material, and cut into a desired shape.

The connector 3 for the connection to a drive source 4 for rotationally driving the torque coil 10, such as a motor, is attached to the proximal end of the shaft main body 1 of the torque coil 10 by a known fixing technique such as epoxy resin or an adhesive.

Note that the disclosed embodiments also provide a shaft for medical instruments in which the torque coil and the drive source are connected by the connector, and a medical instrument including such a shaft. The above-described shaft is especially suitable for a medical instrument including a motor, and is suitably used as a shaft that is provided with an ultrasonic vibrator at the distal end thereof and used in the intravascular ultrasound (IVUS) method, and as a shaft for in-vivo recovery mechanism that is used to remove a substance from a patient's body lumen, for example. The torque coil of the disclosed embodiments particularly exerts the effect in the shaft that rotates at 1000 rpm or higher, or more preferably at 1500 rpm or higher and is used in the intravascular ultrasound (IVUS) method.

In the embodiment, the outer diameter of the inner coil 11 is set to a range of 0.24 to 0.79 mm, and is preferable to be especially in a range of 0.3 to 0.5 mm. The inner diameter of the inner coil 11 is set to a range of 0.17 to 0.57 mm, and is preferable to be especially in a range of 0.2 to 0.4 mm. Moreover, the length in the axial direction of the inner coil 11 is set to a range of 1.0 to 3.0 m. The material of the metal wire 111 forming the inner coil 11 is not especially limited, and austenitic stainless steel such as SUS304 and SUS316, for example, is used.

The inner coil 11 is formed by spirally winding a plurality of metal wires 111 such that no gap is generated between metal wires 111 adjacent to each other in the axial direction of the inner coil 11. In the embodiment, the inner coil 11 is formed by spirally winding 2 to 18 metal wires, and the diameter of each metal wire 111 is set to a range of 0.03 to 0.11 mm. The diameter of each metal wire 111 is preferable to be especially in a range of 0.04 to 0.08 mm. If the inner coil 11 is a single-threaded coil, the rotation performance of the torque coil 10 may be deteriorated.

In the embodiment, the outer diameter of the outer coil 12 is set to a range of 0.3 to 1.0 mm, and is preferable to be especially in a range of 0.4 to 0.6 mm. The inner diameter of the outer coil 12 is set to a range of 0.24 to 0.79 mm, and is preferable to be especially in a range of 0.3 to 0.5 mm. The outer coil 12 is arranged in close contact with the outer periphery of the inner coil 11, and thus the inner diameter of the outer coil 12 is set to be substantially equal to the outer diameter of the inner coil 11. Moreover, the length in the axial direction of the outer coil 12 is equal to that of the inner coil 11, and is set to a range of 1.0 to 3.0 m, for example. The material of the metal wire 121 forming the outer coil 12 is not especially limited, and austenitic stainless steel such as SUS304 and SUS316, for example, is used. The metal wire 111 forming the inner coil 11 and the metal wire 121 forming the outer coil 12 are preferably made of the same material.

The outer coil 12 is formed by spirally winding a plurality of metal wires 121 such that no gap is generated between metal wires 121 adjacent to each other in the axial direction of the outer coil 12. In the embodiment, the outer coil 12 is formed by spirally winding 2 to 18 metal wires, and the diameter of each metal wire 121 is set to a range of 0.03 to 0.50 mm. The diameter of each metal wire 121 is preferable to be especially in a range of 0.03 to 0.08 mm.

In the embodiment, the metal wire 111 forming the inner coil 11 and the metal wire 121 forming the outer coil 12 are both round wire with a substantially circular section. However, the embodiments are not limited thereto, and the metal wire 111 and the metal wire 121 may be round wire with an elliptical section or flat wire with a substantially rectangular section.

In the shaft main body 1, the outer coil 12 is arranged on the outer periphery of the inner coil 11 such that the winding direction of the inner coil 11 and the winding direction of the outer coil 12 are opposite from each other, as illustrated in Fig. 2. In this manner, when the shaft main body 1 is twisted in the circumferential direction being the direction where the diameter of the inner coil 11 is increased, the diameter of the inner coil 11 is increased, and the diameter of the outer coil 12 is decreased.

Here, the torque coil 10 is formed so that, when the shaft main body 1 is twisted in the circumferential direction being the direction where the diameter of the inner coil 11 is increased, the change amount of the diameter of the outer coil 12 is larger than the change amount of the diameter of the inner coil 11. Specifically, as illustrated in Fig. 2, a winding angle α in the longitudinal section direction of the inner coil 11 and a winding angle β in the longitudinal section direction of the outer coil 12 are made different from each other, and the winding angle α in the longitudinal section direction of the inner coil 11 is set to be larger than the winding angle β in the longitudinal section direction of the outer coil 12. In this manner, with the winding angle α of the inner coil 11 larger than the winding angle β of the outer coil 12 (that is, the twisting angle of the inner coil 11 is more upright than the twisting angle of the outer coil 12), the change amount of the diameter of the outer coil 12 is larger than the change amount of the diameter of the inner coil 11 when the shaft main body 1 is twisted in the circumferential direction being the direction where the diameter of the inner coil 11 is increased, as described above.

The relation between the winding angle α in the longitudinal section direction of the inner coil 11 and the winding angle β in the longitudinal section direction of the outer coil 12 is determined depending on the combination of the wire diameter of the metal wire 111 and 121 forming the inner coil 11 and the outer coil 12, respectively, and the set number of wires. For example, if the diameter of the wire forming the coil is fixed, the winding angle in the longitudinal section direction of the coil is decreased as the number of wires of the coil is increased, and is increased as the number of wires of the coil is decreased. Moreover, if the number of wires forming the coil is fixed, the winding angle in the longitudinal section direction of the coil is decreased as the diameter of the wire forming the coil is increased, and is increased as the diameter of the wire forming the coil is decreased.

In the above-described torque coil 10, the winding direction of the inner coil 11 and the winding direction of the outer coil 12 are opposite from each other. Thus, when the torque coil 10 is twisted in the circumferential direction being the direction where the diameter of the inner coil 11 is increased, the diameter of the inner coil 11 is increased, while the diameter of the outer coil 12 is decreased. As a result, the outer coil 12 and the inner coil 11 are pressed against each other. Then, with the change amount of the diameter of the outer coil 12 larger than the change amount of the diameter of the inner coil 11, the outer coil 12 strongly tightens the inner coil 11, so that the layers of the outer coil 12 and the inner coil 11 are brought into firm and close contact with each other. Thus, the twisting rigidity of the torque coil 10 is enhanced, thereby suppressing the occurrence of kinks due to rotational resistance.

The above has described the torque coil (two-layer coil structure) according to the disclosed embodiments with reference to the drawings. However, the disclosed embodiments are not limited to the above-described one, and various changes can be made. For example, the shapes, lengths, diameters, and the like of the inner coil 11 and the outer coil 12 forming the torque coil 10 may be appropriately set in accordance with the use purpose, position, or the like. Moreover, lead wire or the like may be inserted into the shaft main body 1, or a member other than the inner coil 11 and the outer coil 12, such as a reinforcing body or an X-ray impermeable marker, for example, may be provided in the shaft main body 1.

### [Examples]

Hereinafter, the disclosed embodiments will be described more concretely using examples and the like, but the disclosed embodiments are not limited to these examples and the like.

In order to verify that the torque coil of the disclosed embodiment has excellent twisting rigidity, there were produced a plurality of testing torque coils each including an inner coil that is a multi-threaded coil formed by spirally winding a plurality of metal wires and an outer coil that is a multi-threaded coil arranged in close contact with the outer periphery of the inner coil and formed by spirally winding a plurality of metal wires, and the maximum torque force and the twisting rigidity were measured using a motor and a torque sensor.

A plurality of testing torque coils were produced while changing the diameter and number of wires of the inner coil and the outer coil. All of the testing torque coils were produced such that the inner diameter is 0.32 mm and the length is 1606 mm. The structures of the inner coil and the outer coil of the produced testing torque coils are shown in Table 1.

**[Table 1]**

| No. | Inner coil | | | | Outer coil | | | |
|---|---|---|---|---|---|---|---|---|
| | Wire diameter [mm] | Number of wires | Pitch [mm] | Twisting angle [° ] | Wire diameter [mm] | Number of wires | Pitch [mm] | Twisting angle [° ] |
| Example 1 | 0.03 | 8 | 0.264 | 76.5 | 0.09 | 8 | 0.880 | 58.8 |
| Example 2 | 0.04 | 8 | 0.358 | 72.4 | 0.08 | 8 | 0.762 | 63.0 |
| Example 3 | 0.05 | a | 0.457 | 68.5 | 0.07 | 8 | 0.652 | 67.0 |
| Comparative Example 1 | 0.06 | 8 | 0.565 | 64.7 | 0.06 | 8 | 0.549 | 70.7 |
| Comparative Example 2 | 0.07 | 8 | 0.684 | 61.0 | 0.05 | 8 | 0.450 | 74.3 |
| Comparative Example 3 | 0.08 | 8 | 0.819 | 57.4 | 0.04 | 8 | 0.356 | 77.7 |
| Example 4 | 0.06 | 8 | 0.565 | 64.7 | 0.06 | 16 | 1.320 | 50.0 |
| Example 5 | 0.06 | 8 | 0.565 | 64.7 | 0.06 | 14 | 1.082 | 55.4 |
| Example 6 | 0.06 | 8 | 0.565 | 64.7 | 0.06 | 12 | 0.882 | 60.7 |
| Example 7 | 0.06 | 8 | 0.565 | 64.7 | 0.06 | 10 | 0.707 | 55.8 |
| Comparative Example 4 | 0.06 | 8 | 0.565 | 64.7 | 0.06 | 8 | 0.549 | 70.7 |
| Comparative Example 5 | 0.06 | 10 | 0.748 | 57.9 | 0.06 | 8 | 0.549 | 70.7 |
| Comparative Example 5 | 0.06 | 12 | 0.973 | 50.8 | 0.06 | a | 0.549 | 70.7 |
| Comparative Example 7 | 0.06 | 14 | 1.275 | 43.1 | 0.06 | 8 | 0.549 | 70.7 |

In the examples 1 to 3 and the comparative examples 1 to 3 in Table 1, the wire diameters of the inner coil and the outer coil are changed while the number of wires of the inner coil and the outer coil is fixed to eight. Meanwhile, in the examples 4 to 7 and the comparative examples 4 to 7 in Table 1, the number of wires of the inner coil and the outer coil is changed while the wire diameters of the inner coil and the outer coil are fixed to 0.06 mm. Note that the pitch in Table 1 indicates a length of one cycle in the coil axis direction where the wire is wound.

Next, assuming the case where one end of each of the produced testing torque coils of the examples and comparative examples is fixed and the other end is twisted by 360°, there were calculated a change amount d₁ of the diameter of the inner coil when the inner coil having a length of 1596 mm is twisted by 360° in the circumferential direction being the direction where the diameter of the inner coil is increased and a change amount d₂ of the diameter of the outer coil when the outer coil having a length of 1596 mm is twisted by 360° in the circumferential direction being the direction where the diameter of the outer coil is decreased. The measurement results are shown in Table 2.

Note that the reason why the theoretical change amount d₁ of the diameter of the inner coil and change amount d₂ of the diameter of the outer coil were calculated with a premise that the length of each coil is 1596 mm although the length of the actually produced testing torque coil is 1606 mm is because both ends of the testing torque coil is attached to a motor and a torque sensor to measure a maximum torque force and twisting rigidity for each of the produced testing torque coils of the examples and comparative examples, as described later, and 5 mm of each of the both ends is chucked to a connector for attachment to the motor and the torque, which makes the length of the testing torque coil subjected to measurement of the maximum torque force and twisting rigidity 1596 mm.

**[Table 2]**

| No. | Inner coil | | | | | | Outer coil | | | | | | d1/d2 |
|---|---|---|---|---|---|---|---|---|---|---|---|---|---|
| | PCD**π* | Pitch number | Twisting degree/P | PCD**π* after twisting | PCD after twisting | Radial direction change amount d₁ [mm] | PCD**π* | Pitch number | Twisting degree/P | PCD**π* after twisting | PCD after twisting | Radial direction change amount d₂ [mm] | |
| Example 1 | 1.0996 | 6,055 | 0.06 | 1.0997 | 0.3501 | 0.00006 | 1.4765 | 1.814 | 0.20 | 1.4774 | 0.4703 | -0.00026 | -4.48094 |
| Example 2 | 1.1310 | 4,463 | 0.08 | 1.1312 | 0.3601 | 0.00008 | 1.5080 | 2,096 | 0.17 | 1.5087 | 0.4802 | 0.00023 | -2.83898 |
| Example 3 | 1.1624 | 3.489 | 0.10 | 1.1627 | 0.3701 | 0.00011 | 1.5394 | 2,449 | 0.15 | 1.5400 | 0.4902 | -0.00020 | -1.88690 |
| Comparative Example 1 | 1.1938 | 2,823 | 0.13 | 1.1942 | 0.3801 | 0.00013 | 1.5708 | 2,909 | 0.12 | 1.5713 | 0.5002 | -0.00017 | -1.27689 |
| Comparative Example 2 | 1.2252 | 2.332 | 0.15 | 1.2257 | 0.3902 | 0.00017 | 1.6022 | 3.543 | 0.10 | 1.6027 | 0.5101 | -0.00014 | -0.86089 |
| Comparative Example 3 | 1.2566 | 1,949 | 0.18 | 1.2573 | 0.4002 | 0.00021 | 1.6336 | 4.480 | 0.08 | 1.6340 | 0.5201 | -0.00012 | -0.56561 |
| Example 4 | 1.1938 | 2,823 | 0.13 | 1.1942 | 0.3801 | ' 0.00013 | 1.5708 | 1.209 | 0.30 | 1.5721 | 0.5004 | -0.00041 | -3.07214 |
| Example 5 | 1.1938 | 2,823 | 0.13 | 1.1942 | 0.3801 | 0.00013 | 1.5708 | 1,475 | 0.24 | 1.5719 | 0.5003 | -0.00034 | -2.51795 |
| Example 6 | 1.1938 | 2,823 | 0.13 | 1.1942 | 0.3801 | 0.00013 | 1.5708 | 1,810 | 0.20 | 1.5717 | 0.5003 | -0.00028 | -2.05198 |
| Example 7 | 1.1938 | 2,823 | 0.13 | 1.1942 | 0.3801 | 0.00013 | 1.5708 | 2,259 | 0.16 | 1.5715 | 0.5002 | -0.00022 | -1.64446 |
| Comparative Example 4 | 1.1938 | 2.823 | 0.13 | 1.1942 | 0.3801 | 0.00013 | 1.5708 | 2.909 | 0.12 | 1.5713 | 0.5002 | -0.00017 | - 1.27689 |
| Comparative Example 5 | 1.1938 | 2.133 | 0.17 | 1.1944 | 0.3802 | 0.00018 | 1.5708 | 2,909 | 0.12 | 1.5713 | 0.5002 | -0.00017 | -0.96452 |
| Comparative Example 6 | 1.1938 | 1,640 | 0.22 | 1.1945 | 0.3802 | 0.00023 | 1.5708 | 2.909 | 0.12 | 1.5713 | 0.5002 | -0.00017 | -0.74160 |
| Comparative Example 7 | 1.1938 | 1,252 | 0.29 | 1.1948 | 0.3803 | 0.00030 | 1.5708 | 2.909 | 0.12 | 1.5713 | 0.5002 | -0.00017 | -0.56625 |

The theoretical change amount d₁ of the diameter of the inner coil and change amount d₂ of the diameter of the outer coil in each of the examples and comparative examples, and ratios of the change amount d₁ of the diameter of the inner coil and change amount d₂ of the diameter of the outer coil (d₁/d₂), which are shown in Table 2, were calculated in the following method.
(1) The PCD of each of the inner coil and the outer coil was found. Here, a PCD is a diameter of a virtual circle with a center of the coil as a center in a transverse section view of the coil, the virtual circle passing the center in the radial direction of each wire forming the coil, and is a value calculated by "coil inner diameter + wire length in radial direction".
(2) The periphery of the circle with a PCD as a diameter was found by "PCD × π" for each of the inner coil and the outer coil.
(3) The twisting degree was found by "twisting angle (360° in the examples)/pitch number" for each of the inner coil and the outer coil. Note that the pitch number is found by "coil length/length of one pitch". The length of one pitch was calculated by averaging 10 pitches measured actually.
(4) For the inner coil, the periphery of the circle with a PCD after twisting in the diameter increasing direction as a diameter was found by "PCDπ + ({twisting degree/360°} × PCDπ)", and the periphery was divided by π to calculate a PCD after twisting in the diameter increasing direction.
(5) For the outer coil, the periphery of the circle with a PCD after twisting in the diameter decreasing direction as a diameter was found by "PCDπ - ({twisting degree/360°} × PCDπ)", and the periphery was divided by π to calculate a PCD after twisting in the diameter decreasing direction.
(6) The "PCD before twisting the inner coil" is deducted from the "PCD after twisting the inner coil in the diameter increasing direction" to find a "change amount d₁ of the diameter of the inner coil", while the "PCD before twisting the outer coil" is deducted from the "PCD after twisting the outer coil in the diameter decreasing direction" to find a "change amount d₂ of the diameter of the outer coil".
(7) With these, the ratio (d₁/d₂) of the change amount d₁ of the diameter of the inner coil and the change amount d₂ of the diameter of the outer coil (d₁/d₂) was calculated.
Note that the twisting angle was set to 360° in the examples but in a case where the length of the inner coil and the outer coil is smaller than 1596 mm, the above-described d₁/d₂ may be found with the twisting angle calculated by "360[°]×{(l ength of inner coil and outer coil [mm])/1596 [mm]}".

Subsequently, the produced testing torque coil of each of the examples and comparative examples was attached to a guide wire transmission characteristics measuring device PT-1950GHS (by PROTEC) including a motor and a torque sensor, and rotated in a forward direction by the motor and twisted so as to measure the maximum torque force and twisting rigidity of the testing torque coil of each of the examples and comparative examples. The measurement was performed a plurality of times for each of the examples and comparative examples, and the average value was calculated as a measurement result. The measurement results are shown in Table 3.

**[Table 3]**

| No. | d1/d2 | Maximum torque force [mN · m] | Twisting rigidity [mN · m/deg] |
|---|---|---|---|
| Example 1 | -4.48094 | 2.995 | 0.001198 |
| Example 2 | -2.83898 | 4.168 | 0.001502 |
| Example 3 | -1.88690 | 2.409 | 0.001416 |
| Comparative Example 1 | -1.27689 | 0.944 | 0.000772 |
| Comparative Example 2 | -0.86089 | 0.872 | 0.000633 |
| Comparative Example 3 | -0.56561 | 0.907 | 0.000665 |
| Example 4 | -3.07214 | 12.874 | 0.001813 |
| Example 5 | -2.51795 | 7.941 | 0.001584 |
| Example 6 | -2.05198 | 5.029 | 0.001919 |
| Example 7 | -1.64446 | 2.269 | 0.001500 |
| Comparative Example 4 | -1.27689 | 0.944 | 0.000772 |
| Comparative Example 5 | -0.96452 | 0.970 | 0.000794 |
| Comparative Example 6 | -0.74160 | 0.630 | 0.000650 |
| Comparative Example 7 | -0.56625 | 0.494 | 0.000386 |

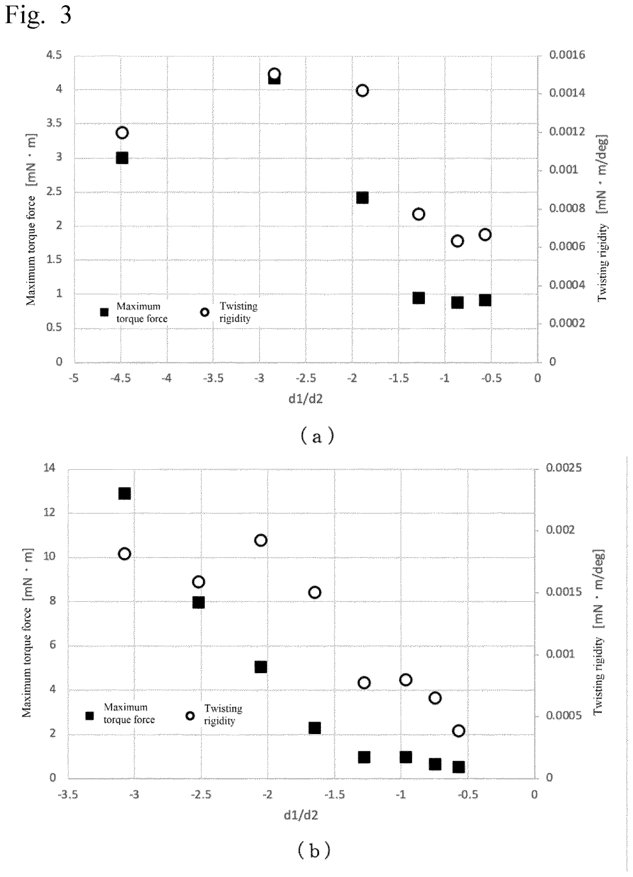

Fig. 3 is a graph illustrating the relation between the ratio of change amounts in a radial direction of the inner coil and the outer coil and the maximum torque force and the twisting rigidity in the torque coils of the examples 1 to 7 and comparative examples 1 to 7 on the basis of the measurement results shown in Table 3. Fig. 3(a) is a graph regarding the examples 1 to 3 and comparative examples 1 to 3, and Fig. 3(b) is a graph regarding the examples 4 to 7 and comparative examples 4 to 7.

As is clear from Fig. 3, the torque coils of the examples 1 to 7 have more excellent twisting rigidity than the torque coils of the comparative examples 1 to 7. Especially in a case where the change amount d₁ of the diameter of the inner coil when the inner coil having a length of 1596 mm is twisted by 360° in the circumferential direction being the direction where the diameter of the inner coil is increased, and the change amount d₂ of the diameter of the outer coil when the outer coil having a length of 1596 mm is twisted by 360° in the circumferential direction being the direction where the diameter of the outer coil is decreased, satisfy the relation of -4.5 < d₁/d₂ < -1.6, the maximum torque force of the torque coil (two-layer coil structure) is improved, thereby achieving a torque coil having more excellent twisting rigidity.

It is understood that especially in the torque coil configured such that the diameters of wires of the inner coil and the outer coil are same and the number of wires of the outer coil is larger than that of the inner coil, such as the torque coils of the examples 4 to 7, the twisting rigidity is extremely excellent, and the maximum torque force is also significantly improved.

### [Reference Signs List]

- 10: torque coil (two-layer coil structure)
1 shaft main body
11 inner coil
12 outer coil
2 housing
3 connector
4 motor

## Claims

1. A two-layer coil structure, comprising:
an inner coil that is formed by spirally winding metal wire; and
an outer coil that is arranged to be in close contact with an outer periphery of the inner coil and formed by spirally winding metal wire, wherein
a winding direction of the inner coil and a winding direction of the outer coil are opposite from each other,
when the two-layer coil structure is twisted in a circumferential direction of the two-layer coil structure being the direction where a diameter of the inner coil is increased, a change amount of the diameter of the inner coil is smaller than a change amount of a diameter of the outer coil,
a length 1596 mm is set as one unit of each of the inner coil and the outer coil, and the change amount d₁ of the diameter of the inner coil when the inner coil of the one unit length is twisted by 360° in the circumferential direction being the direction where the diameter of the inner coil is increased, and the change amount d₂ of the diameter of the outer coil when the outer coil of the one unit length is twisted by 360° in the circumferential direction being the direction where the diameter of the outer coil is decreased, satisfy the relation of -4.5 < d₁/d₂ < -1.6.

2. The two-layer coil structure according to claim 1, wherein the inner coil is formed by spirally winding 2 to 18 metal wires.
